# EUROPEAN PATENT APPLICATION

(11) **EP 2 090 663 A2**
(43) Date of publication of application: **19.08.2009**
(21) Application number: 08075845.1
(22) Date of filing: 27.06.2001
(51) Int. Cl.: C12Q 1/02, C12N 5/06, G01N 33/50

(54) **Method of preparing heart muscle cells and method of searching for remedy for heart diseases**

(30) Priority: 28.06.2000 JP 2000194805
(62) Divisional of application: 01945643.3
(71) Applicant: Takeda Pharmaceutical Company Limited, Osaka-shi Osaka 541 (JP)
(72) Inventor: Kimura, Haruhide, Ibaraki 300-2655 (JP); Koyama, Nobuyuki, Osaka 532-8686 (JP); Tanida, Seiichi, Kyoto 617-0857 (JP); Kishimoto, Tadamitsu, Osaka 584-0021 (JP); Takihara, Keiko, Osaka 534-0027 (JP); Hiroto, Hisao, Hyogo 665-0845 (JP)
(74) Representative: Weiss, Wolfgang

(57) **Abstract**

The present invention relates to a method of preparing primary heart muscle cells, characterized by washing a fine fragmented heart tissue with a phosphate-buffered physiological saline to thereby eliminate non-heart muscle cells and then hemolyzing the heart tissue digested with a protease to thereby eliminate erythrocytes. According to this method, highly pure primary heart muscle cells can be conveniently obtained in a large amount from the heart of an animal embryo or newborn. By using the heart muscle thus prepared, apoptosis of heart muscle cells can be efficiently and highly sensitively detected. Thus, it is possible to efficiently screen candidate compounds for heart muscle cell apoptosis inhibitors, gp 130-mediated receptor agonists, heart muscle cell-protective signal enhancers, preventives and remedies for heart diseases. A method of detecting apoptosis comprises inducing apoptosis of heart muscle cells by incubating the cells in a serum-free medium, adding serum to the liquid culture medium thereof, then incubating the cells and counting viable heart muscle cells.

## Description

### TECHNICAL FIELD

This invention relates to a method of preparing highly pure primary heart muscle cells easily in a large amount, which is useful for searching for remedies for heart diseases having an inhibitory action on apoptosis of heart muscle cells or an enhancing action on heart muscle cell-protective signals, as well as a method of searching for such remedies efficiently and highly sensitively by dealing with a large number of samples.

This invention relates in particular to a method of searching for remedies for heart diseases, characterized by preparing highly pure primary-culture of heart muscle cells easily in a large amount from the heart of an animal embryo or a newborn and detecting heart muscle cell apoptosis efficiently and highly sensitively by using the prepared heart muscle cells, in order to search for prophylactic and therapeutic agents for heart diseases having an inhibitory action on apoptosis of heart muscle cells or an enhancing action on heart muscle cell-protective signals by using the highly pure primary heart muscle cells.

### BACKGROUND ART

Apoptosis is concerned closely with morphology and histogenesis in the development process, maintenance of homeostasis, and protection of the living body, and refers to cell death having an important role in maintaining the life of individuals. When the process of death regulated by genes is congenitally or postnatally hindered, apoptosis is excessively induced or inhibited to cause functional disorders in various organs, leading to diseases (Shin Yonehara, Saishin Igaku (Latest Medicine), vol. 54, p. 825, 1999). Accordingly, there is demand for an understanding of diseases from the viewpoint of apoptosis and for development of diagnostic methods or remedies based on the molecular mechanism.

Apoptosis is opposite to cell proliferation. Accordingly, heart muscle cells, that is, finally differentiated cells not growing, are considered less related to apoptosis. However, it was revealed in recent years that apoptosis is concerned closely with onset or progress of various heart diseases (R. Sanders Williams, The New England Journal of Medicine, vol. 341, p. 759, 1999). Heart muscle cells are not proliferated so that when heart muscle cells disappear by apoptosis, the contracting function should be maintained by surviving cells only. Accordingly, disappearance of heart muscle cells beyond the necessary threshold for maintaining the contracting function of the heart would result in abnormal heart functions, leading to diseases. Actually, apoptosis of heart muscle cells is observed in various animal models with cardiac insufficiency or in patients with cardiac insufficiency, and it is noted that disappearance and dropout of heart muscle cells by apoptosis may be concerned with onset and progress of cardiac insufficiency (Narula, J. et al., The New England Journal of Medicine, vol. 335, p. 1182, 1996). It is further recognized that in heart muscle cells in patients with cardiac insufficiency, an apoptosis-inhibitory factor Bcl-2 is expressed in excess, which is shown to be a possible mechanism for compensating for cardiac insufficiency (Olivetti, G. et al., The New England Journal of Medicine, vol. 336, p. 1131, 1997), and serum levels of soluble Fas (sFas has an inhibitory activity on apoptosis) deficient in a membrane penetration domain, secreted due to splicing of a Fas receptor known to induce apoptosis, are increased significantly in proportion to severeness in NYHA class (New York Heart Association Functional Class) but independently of fundamental diseases, and thus an increase in serum levels of sFas is considered to be a compensatory mechanism for inhibiting promotion of apoptosis at the time of cardiac insufficiency (Nishigaki, K. et al., Journal of the American College of Cardiology, vol. 29, p. 1214, 1997), and it is known that in the heart with dilation type myocardiosis, deoxyribonuclease I (DNase I) considered as one indicator of apoptosis is contained in quantity 7 or more times greater than in healthy persons (Yao, M. et al., Journal of Molecular & Cell Cardiology, vol. 28, p. 95, 1996).

On one hand, recent important findings related to protection of heart muscle cells include those from studies on a mouse deficient in gp130 specifically in the ventricle. By analysis of this mouse, it was revealed that signals (gp130 signals) from gp130-mediated receptors play an important role in protecting the functions of the heart, and this study is noted as a new development of heart muscle cell-protective signals leading to therapy of heart diseases (Hirota, et al., Cell, vol. 97, p. 189, 1999, and Senior, K. Molecular Medicine Today, vol. 5, p. 283, 1999).

It is strongly possible that compounds having an inhibitory action on apoptosis of heart muscle cells or an enhancing action on heart muscle cell-protective signals can serve as new remedies for heart diseases.

For searching for compounds having an inhibitory action on apoptosis of heart muscle cells, compounds having an agonistic action on gp130-mediated receptors or compounds having an enhancing action on heart muscle cell-protective signals, it is necessary to rapidly and efficiently screen compounds inhibiting apoptosis of heart muscle cells from a large number of samples. It would be considered best to screen such compounds not by using animals themselves or heart tissue but by using primary heart muscle cells prepared easily in a large amount.

At present, heart muscle cells are prepared by a method of isolating the cells by digesting mainly animal hearts with a protease such as trypsin or collagenase. In this method, digestion with a protease such as trypsin or collagenase is carried out generally after the heart of an animal embryo or a newborn is fragmented to facilitate digestion with the protease (Kiyota Goto & Hiroyuki Kaneko, Shinzo Kekkan Kenkyu Hoho No Kaihatsu (Development in Method of Studying Heart and Blood Vessels) edited by Setsuro Ebashi, page 3, Japan Scientific Societies Press (JSSP) (1983)). In this method, however, there was a problem that blood cannot be sufficiently removed so that owing to contamination with blood-derived cells, the purity of the resultant heart muscle cells is lowered. To solve this problem, a method of recovering highly pure heart muscle cells by means of Ficoll etc. (Jojo, K. et al., Biochemical and Biophysical Research Communications, vol. 225, p. 340 (1996)) and a method of selectively inhibiting proliferation of non-heart muscle cells by adding a chemical such as a DNA synthesis inhibitor (bromodeoxyuridine (BrDU)) (Chacko, B. et al., Journal of Cell Biology, vol. 55, p. 36A (1972)) or calcium ionophores (A23187) (Kaneko, H. et al., Experimental Cell Research, vol. 142, p. 407 (1982)) have been devised. However, the method of using Ficoll etc. is time-consuming for recovery and the method of using BrDU or A23187 has a problem in recovery of the cells and influence of BrDU or A23187 on heart muscle cells because the chemical itself is toxic to cells. It is difficult to select compounds inhibiting apoptosis of heart muscle cells rapidly and efficiently by using heart muscle cells prepared in such preparative methods because of the above problems, and therefore these preparative methods are not preferable as the methods of preparing heart muscle cells for dealing with a large number samples in order to permit compounds inhibiting apoptosis of heart muscle cells to be rapidly and efficiently selected from a large number of samples.

Another known method of preparing heart muscle cells involves excising heart muscle cells from a matured animal, setting the cells in a Langendolff type perfusion device, washing blood away and perfusing a solution of a protease such as trypsin or collagenase (Haruo Sugi & Yukio Hiramoto: Jikken Seibutsugaku Koza (Lecture on Experimental Biology), vol. 10, p. 27 (1984)). However, this method requires the special device, and also has problems such as very low survival of heart muscle cells from a matured animal, difficulty in technical skills, and the number and properties of heart muscle cells recovered, so that similar to the preparative methods described above, this method is not suitable for search by dealing with a large number of samples.

As described above, it was necessary to develop a new method of preparing primary heart muscle cells easily in a large amount in order to permit compounds inhibiting heart muscle cell apoptosis to be selected rapidly and efficiently from a large number of samples.

For searching for compounds having an inhibitory action on apoptosis of heart muscle cells, compounds having an agonistic action on gp130-mediated receptors or compounds having an enhancing action on heart muscle cell-protective signals, not only the method of preparing highly pure primary heart muscle cells, but also an easy method for detecting apoptosis of heart muscle cells, suitable for searching for the compounds are required.

For inducing apoptosis of primary-culture heart muscle cells by removing serum from a culture, the density of the cells should be reduced to a certain degree because it is considered that at higher density, cells are contacted with one another to induce protective signals and to produce protection factors, and further the activity of intracellular dehydrogenase activity in viable heart muscle cells in a serum-free state is low, and therefore it is difficult to detect apoptosis of heart muscle cells quantitatively by e.g. a method of reduction of MTT (3-(4,5-dimethylthiazol-2-yl)-2,5-diphenyltetrazolium bromide) (Tada et al., Journal of Immunological Methods), vol. 93, p. 157 (1986)). For detecting apoptosis of heart muscle cells, a method of staining dead cells with trypan blue etc. or a method of adding MTT to form crystals of formazan in viable cells and then counting the number of the viable cells has been used (Shenz, Z. et al., The Journal of Biological Chemistry, vol. 272, No. 9, p. 5783 (1997)). However, it is difficult to deal with a large number of samples in such methods, and for searching for compounds having an inhibitory action on apoptosis of heart muscle cells, compounds having an agonistic action on gp130-mediated receptors or compounds having an enhancing action on heart muscle cell-protective signals, it was necessary to devise a method of detecting apoptosis of heart muscle cells more efficiently and highly sensitively.

### DISCLOSURE OF THE INVENTION

As a result of extensive study for solving the problem described above, the present inventors found that fragmented heart tissue of an animal embryo or a newborn is washed with a phosphate-buffered physiological saline before treatment with a protease such as trypsin or collagenase, and residual erythrocytes are removed by a low-isotonic hemolysis procedure, whereby highly pure primary heart muscle cells can be prepared easily in a large amount. Hereinafter, this preparative method is also referred to as the method of preparing heart muscle cells according to this invention.

The present inventors also found a method of efficiently and sensitively detecting apoptosis of heart muscle cells induced by removing serum from a culture medium, which comprises adding serum to the culture, culturing the cells for about 17 hours to revitalize the cells, and then determining the number of viable cells by using a cell counting kit-8 (a product of Dojin Kagaku Kenkyusho) with WST-8 (2-(2-methoxy-4-nitrophenyl)-3-(4-nitrophenyl)-5-(2,4-disulfophenyl)-2H-tetrazolium, monosodium salt) as a coloring substrate. Hereinafter, this method is also referred to as the method of detecting apoptosis according to this invention.

As a result of further investigation using a combination of these methods, the present inventors achieved the present invention.

Thus, the present invention relates to [1] a method of easily preparing a large amount of highly pure primary-culture heart muscle cells useful for searching for compounds having an inhibitory action on apoptosis of heart muscle cells, compounds having an agonistic action on gp130-mediated receptors or compounds having an enhancing action on heart muscle cell-protective signals, [2] a method of detecting these compounds efficiently and highly sensitively, [3] a searching method (screening method) for dealing with a large number of samples, which comprises the above preparative method in combination with the above detection method, and [4] heart muscle cell apoptosis inhibitors, gp130-mediated receptor agonists, heart muscle cell-protective signal enhancers, or prophylactic and/or therapeutic agents for heart diseases, comprising compounds obtained by the searching method or salts thereof.

This invention relates in particular to:
(1) a method of preparing primary heart muscle cells, characterized in that after non-heart muscle cells are removed from fragmented heart tissue, the heart tissue is digested with a protease,
(2) the preparative method according to the above-mentioned (1), characterized in that the non-heart muscle cells are removed by washing the fragmented heart tissue with a phosphate-buffered physiological saline,
(3) a method of preparing primary heart muscle cells, characterized in that after fragmented heart tissue is digested with a protease, erythrocytes are removed,
(4) the preparative method according to the above-mentioned (3), characterized in that erythrocytes are removed by hemolysis treatment,
(5) a method of preparing primary heart muscle cells, characterized by hemolysis treatment of heart muscle cells isolated from heart tissue,
(6) a method of preparing primary heart muscle cells, characterized in that after non-heart muscle cells are removed from fragmented heart tissue, the heart tissue is digested with a protease, and then erythrocytes are removed,
(7) the preparative method according to the above-mentioned (6), characterized in that the fragmented heart tissue is washed with a phosphate-buffered physiological saline to remove non-heart muscle cells, and the heart tissue digested with a protease is subjected to hemolysis treatment to remove erythrocytes,
(8) a method of detecting apoptosis of heart muscle cells, characterized in that after serum is added to a culture of heart muscle cells having apoptosis induced by culturing the cells in a serum-free medium, the cells are cultured, and the number of viable heart muscle cells is determined,
(9) the detection method according to the above-mentioned (8), wherein the number of viable heart muscle cells is determined by measuring an intracellular dehydrogenase activity,
(10) the detection method according to the above-mentioned (8), wherein the culture time after addition of serum is about 6 to 30 hours,
(11) the detection method according to the above-mentioned (9), wherein the intracellular dehydrogenase activity is measured by using MTT or WST-8,
(12) the detection method according to the above-mentioned (8), wherein the primary heart muscle cells obtained by the preparative method described in any one of the above-mentioned (1) to (7) are cultured in a serum-free medium,
(13) a method of screening a compound inhibiting apoptosis or a salt thereof, characterized in that by the detection method described in the above-mentioned (12), the number of viable cells in a case (i) where apoptosis is induced in the presence of a test compound is compared with that in a case (ii) where apoptosis is induced in the absence of a test compound,
(14) an inhibitor of apoptosis of heart muscle cells, comprising a compound obtained by the screening method described in the above-mentioned (13) or a salt thereof,
(15) a gp130-mediated receptor agonist, comprising a compound obtained by the screening method described in the above-mentioned (13) or a salt thereof,
(16) an enhancer of heart muscle cell-protective signal, comprising a compound obtained by the screening method described in the above-mentioned (13) or a salt thereof,
(17) a prophylactic and/or therapeutic agent for heart diseases, comprising the heart muscle cell apoptosis inhibitor described in the above-mentioned (14), the gp130-mediated receptor agonist described in the above-mentioned (15) or the heart muscle cell-protective signal enhancer described in the above-mentioned (16),
(18) a method of preventing and treating heart diseases, characterized in that an effective dose of the heart muscle cell apoptosis inhibitor described in the above-mentioned (14), the gp130-mediated receptor agonist described in the above-mentioned (15) or the heart muscle cell-protective signal enhancer described in the above-mentioned (16) is administered to mammals, and
(19) use of the heart muscle cell apoptosis inhibitor described in the above-mentioned (14), the gp130-mediated receptor agonist described in the above-mentioned (15) or the heart muscle cell-protective signal enhancer described in the above-mentioned (16) for producing a prophylactic and/or therapeutic agent for heart diseases.

### BRIEF DESCRIPTION OF THE DRAWINGS

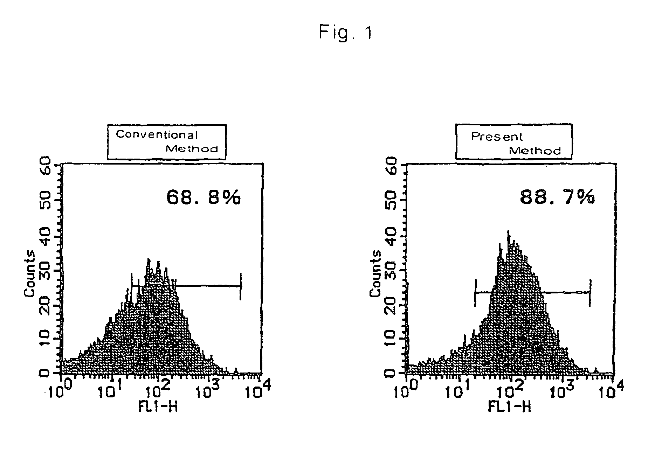

Fig. 1 shows the results of comparison in purity between heart muscle cells obtained by the method of preparing primary heart muscle cells according to this invention, and heart muscle cells obtained in a conventional method, the comparison which was carried out in Example 1. In Fig. 1, the "conventional method" and the "present method" show the results of flow cyometric analysis of the heart muscle cells obtained by the conventional method and the method of preparing heart muscle cells according to this invention, respectively.

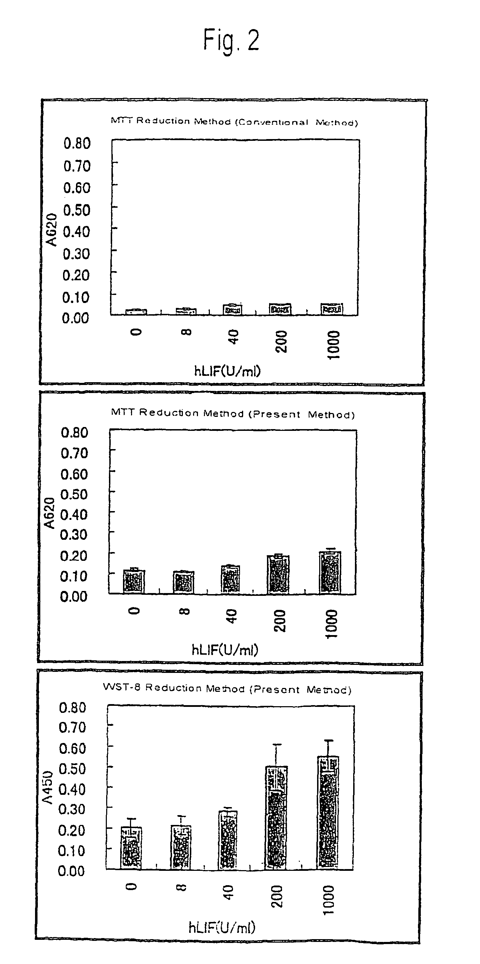

Fig. 2 shows the results of comparison in the detection sensitivity of inhibitory action of hLIF on apoptosis of heart muscle cells between the present method of detecting apoptosis and the conventional method. In Fig. 2, the "MTT reduction method (conventional method)" shows the results of the measurement by the conventional method with MTT as the coloration substrate, the "MTT reduction method (present method)" shows the results of the measurement by the present method of detecting apoptosis with MTT as the coloration substrate, and the "WST-8 reduction method (present method)" shows the results of the measurement by the present method of detecting apoptosis with WST-8 as the coloration substrate.

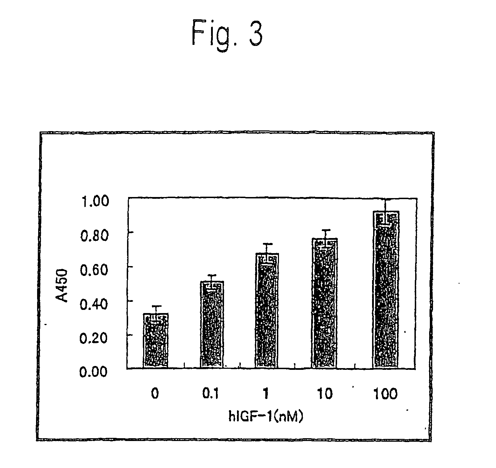

Fig. 3 shows the results of detection, by the present method, of the inhibitory activity of hIGF-1 on apoptosis of heart muscle cells, which was carried out in Example 4.

### BEST MODE FOR CARRYING OUT THE INVENTION

Hereinafter, this invention is described in more detail.

### (1) Method of preparing heart muscle cells according to this invention

This method of the present invention is suited for, but is not limited to, preparation of a large amount of heart muscle cells from the heart of an animal, e.g. rat or mouse, embryo or a newborn, from which blood is hardly washed away with a Langendolff type perfusion device or the like.

In the case of a rat newborn, for example, the heart is excised, and the excised heart is washed with a phosphate-buffered physiological saline and then fragmented. The fragmented heart tissue is washed with 4 to 5 times with a phosphate-buffered physiological saline or the like, whereby non-heart muscle cells derived from blood can be efficiently removed.

Then, this heart tissue fragments are digested with a protease such as trypsin or collagenase to separate the cells. The conditions such as the type of enzyme, the reaction temperature and the reaction time can be suitably selected depending on the type of animal. The enzyme reaction can be terminated by adding a serum-containing medium. After the reaction is terminated, the cells are collected by centrifuging the cell-containing suspension, and then suspended in a suitable medium. After the cell mass is removed, the cell-containing medium is placed in a Petri dish and cultured for 1 hour.

Under these conditions, many heart muscle cells are still in a floating state, but a majority of non-heart muscle cells such as fibroblast-like cells adhere to the Petri dish, and thus the non-heart muscle cells adhering to the Petri dish can be removed by recovering the floating cells. At this stage, blood-derived cells and a majority of erythrocytes have already been removed in the method of preparing heart muscle cells according to this invention, so that the efficiency of adhesion of non-heart muscle cells such as fibroblast-like cells to the Petri dish is raised, and as a result, the non-heart muscle cells can be efficiently removed. The floating cells are recovered from the Petri dish and subjected to hemolysis with a low-isotonic solution or the like to remove residual erythrocytes. The cells after hemolysis with a low-isotonic solution or the like are suspended in a suitable medium, then placed in a Petri dish and cultured for about 24 hours, whereby the heart muscle cells adhere to the Petri dish.

A majority of cells not adhering at this stage to the Petri dish are damaged heart muscle cells, and these cells can be easily removed by mildly shaking the Petri dish to stir the culture in the Petri dish and exchanging the medium with a fresh one.

In heart muscle cells of low purity obtained by the conventional method, contaminating blood-derived non-heart muscle cells and damaged heart muscle cells are aggregated to adhere to a Petri dish, and thus these cells cannot be sufficiently removed by stirring the Petri dish and exchanging the medium. Accordingly, the plate to which the heart muscle cells adhere should be further washed in order to obtain highly pure heart muscle cells. However, it is difficult to remove these undesirable cells by washing without affecting the heart muscle cells, and a large amount of heart muscle cells necessary for searching by dealing with a large number of samples cannot be obtained in the conventional method.

Now, the method of preparing heart muscle cells according to this invention is described in more detail.

For example, when heart muscle cells of e.g. a rat newborn are prepared, the rat newborn is anesthetized under ether, the abdomen is sterilized and the heart is excised. The excised heart is washed with e.g. a phosphate-buffered physiological saline (pH 7.4) and fragmented with scissors etc. The size of the fragment is preferably 1 mm³ or less. The fragmented heart tissue is washed 4 to 5 times with e.g. a phosphate-buffered physiological saline (pH 7.4) to sufficiently remove non-heart muscle cells derived from blood.

Then, the heart tissue fragments are digested with a protease such as trypsin or collagenase to separate the cells. The conditions such as the type of optimum enzyme, the reaction temperature and the reaction time are varied depending on the type of animal used; for example, when heart muscle cells from rat newborns are used, the cells from 10 newborns are treated for 15 minutes with 5 ml enzyme solution (prepared by dissolving 1.25 mg trypsin (Difco) and 0.25 mg collagenase (Sigma) per ml of PBS), and 2.5 ml enzyme solution is added additionally twice at a 15-minutes interval, and the cells are kept at 37°C under stirring with a stirrer. The enzyme reaction is terminated by adding a medium such as Medium 199 containing serum such as FCS or the like at a final concentration of 10 %. After the reaction is terminated, the cells are collected by centrifuging the cell-containing suspension, and then suspended in a suitable medium such as Medium 199. After the cell mass is removed by filtration with a filter such as a cell strainer (Falcon), the cell-containing medium is placed in a Petri dish and cultured for 1 hour in a CO₂ incubator set at 5 % CO₂, 37°C. At preferable cell density, cells from 10 newborns are suspended in about 50 ml medium.

Then, the floating cells are recovered from the Petri dish and subjected to hemolysis with a low-isotonic solution to remove residual erythrocytes. Specifically, heart muscle cells from 10 newborns are suspended in 2 ml low-isotonic solution (prepared by dissolving 8.29 g NH₄Cl, 1.0 g KHCO₃ and 37 mg EDTA/2Na in 1 L of water) and left for 3 minutes, whereby erythrocytes can be disrupted. The cells after hemolysis with the low-isotonic solution are suspended at a density of about 3×10⁴ cells/well (96-wells plate) in a suitable medium such as Medium 199, then placed in a Petri dish and cultured for about 24 hours in a CO₂ incubator set at 5 % CO₂, 37°C. After culture, the Petri dish is mildly shaken thus stirring the culture in the Petri dish, and then the medium is exchanged with a fresh one, whereby highly pure heart muscle cells are obtained.

### (2) Method of detecting apoptosis according to this invention

The heart muscle cells obtained by the method of the invention are adjusted to a suitable cell density and cultured in a serum-free medium, whereby apoptosis can be induced, and the survival rate of the heart muscle cells after culture for 4 days can be reduced to 50 % or less.

As an index of the viable cells, the activity of an enzyme such as dehydrogenase is low in the viable cells in the serum-free state, thus making it difficult to know the number of the viable cells by the MTT reduction method. According to this invention, on the other hand, a culture of heart muscle cells having apoptosis induced by removing serum is supplemented with serum and further cultured for about 6 to 30 hours (preferably about 17 hours) to increase the dehydrogenase activity of the viable cells, whereby apoptosis of the heart muscle cells can be detected easily and highly sensitively by e.g. a commercial cell counting kit with WST-8 used as the coloration substrate. By using the method of increasing the dehydrogenase activity of viable cells by adding serum to a culture of heart muscle cells having apoptosis induced by removing serum, detection of apoptosis of the heart muscle cells can be carried out by the MTT reduction method etc., but sensitivity can be further increased by using WST-8 as the coloration substrate.

Now, the method of detecting apoptosis according to this invention is described in more detail.

The heart muscle cells obtained by the method of the invention are adjusted to a cell density of 3×10⁴ cells/well (96-wells plate) and cultured in serum-free medium such as Medium 199 in a CO₂ incubator set at 5 % CO₂, 37°C for 4 days, thus inducing apoptosis to reduce the survival rate of the heart muscle cells to 50 % or less.

Serum such as FCS is added at a final concentration of 10 % to the culture, which is then cultured for about 17 hours and measured for its dehydrogenase activity (reduction activity) with MTT or WST-8 as the coloration substrate, to determine the number of viable cells.

According to this invention, a large amount of highly pure primary heart muscle cells can be obtained more easily than by the conventional method. Further, apoptosis of heart muscle cells induced by removing serum from the culture can be detected efficiently and highly sensitively. The method of the invention can be applied not only to heart muscle cells derived from rats but also to those derived from mammals such as mice, guinea pigs, rabbits, dogs etc.

### (3) Screening of candidate compounds for prophylactic and/or therapeutic agents for heart diseases

The method of detecting apoptosis according to this invention is performed by using the heart muscle cells obtained by the method of preparing heart muscle cells according to this invention, whereby compounds inhibiting apoptosis or salts thereof can be screened.

Specifically, compounds inhibiting apoptosis or salts thereof can be screened by comparing the number of viable cells in the case (i) where apoptosis of the heart muscle cells obtained by the present method of preparing heart muscle cells is induced in the presence of a test compound, with that in the case (ii) where apoptosis of the heart muscle cells is induced in the absence of a test compound. Hereinafter, this screening method is also referred to as the screening method of the invention.

Specifically, screening can be carried out by the method described in the Examples described later or by its analogous method.

The compounds obtained by the screening method or salts thereof have an inhibitory action on apoptosis of heart muscle cells, an agonistic action on gp130-mediated receptors and/or an enhancing action on heart muscle cell-protective signals, and can thus be used as prophylactic and/or therapeutic agents for heart diseases (e.g., congestive myocardiopathy, hypertrophic myocardiopathy with obstruction, hypertrophic myocardiopathy without obstruction, idiopathic myocardiopathy, myocardial infarction, chronic cardiac insufficiency etc.).

Test compounds include, for example, peptides, proteins, non-peptide compounds, synthetic compounds, fermentation products, cell extracts, plant extracts and animal tissue extracts, which may be novel or known compounds.

For example, a test compound showing inhibition by about 20 % or more, preferably 30 % or more, more preferably about 50 % or more in terms of the number of viable cells in the case (i), as compared to that in the case (ii), can be selected as a compound inhibiting apoptosis of heart muscle cells.

The compounds obtained by the screening method of the invention include 2-(2-pyridyl)-4H-1,3-benzothiazin-4-one (Compound 1) etc.

The compounds obtained by the screening method of the invention or salts thereof are selected from the above-mentioned test compounds, e.g. peptides, proteins, non-peptide compounds, synthetic compounds, fermentation products, cell extracts, plant extracts, animal tissue extracts and plasma, and have an inhibitory activity on apoptosis of heart muscle cells.

The salts of the compounds include pharmaceutically acceptable salts with acids (inorganic acids, organic acids) or bases (inorganic bases, organic bases).

The compounds obtained by the screening method of the invention or salts thereof can be used orally for example in the form of tablets which may be coated with sugar, capsules, elixirs, microcapsules etc., or parenterally in the form of injections such as a sterile solution and a suspension in water or with other pharmaceutically acceptable liquid. For example, these preparations can be manufactured by mixing the compound or a salt thereof with a physiologically acceptable carrier, a flavoring agent, an excipient, a vehicle, an antiseptic agent, a stabilizer, a binder, etc. in a unit dosage form required in a generally accepted manner that is applied to making pharmaceutical preparations. The effective component in the preparation is controlled in such a dose that an appropriate dose is obtained within the specified range given.

Additives miscible with tablets, capsules, etc. include a binder such as gelatin, corn starch, tragacanth gum, and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, Gaultheria oil and cherry. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition.

Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol and polyethylene glycol), a nonionic surfactant (e.g., polysorbate 80^{™} and HCO-50), etc. Examples of the oily medium include sesame oil and soybean oil, which may also be used in combination with a dissolution aid such as benzyl benzoate and benzyl alcohol. The injection may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The prepared injection is normally filled in an appropriate ampoule.

Since the thus obtained pharmaceutical preparation is safe and low toxic, the preparation can be administered into mammals (e.g., humans, rats, mice, guinea pigs, rabbits, birds, sheep, swine, bovine, horses, cats, dogs, monkeys, etc.).

The dose of the obtained compound or a salt thereof varies depending on the intended disease, subject for administration, etc. In administration of the compound or a salt thereof as a therapeutic or prophylactic agent for myocardial infarction, the compound or a salt thereof is administered generally into an adult (weighing 60 kg) in a daily dose of about 0.1 to 100 mg, preferably about 1.0 to 50 mg, more preferably about 1.0 to 20 mg.

Hereinafter, this invention is described in more detail by reference to the Examples. These examples are a mere illustration of this invention, and this invention is not limited thereto.

### Example 1: Comparison in Number of Cells and Purity between Heart Muscle Cells Obtained by the Method of the Invention and Heart Muscle Cells Obtained by Conventional Method

In this experiment, Wister rat newborns (within 1 day after birth) available from Charles River were used. The rat newborns were anesthetized under ether, their abdomens were sterilized with 70 % ethanol and their hearts were excised with tweezers. The excised heart was washed with a phosphate-buffered physiological saline (T900 produced by Takara) and fragmented with surgical scissors.

In the conventional method (Kiyota Goto & Hiroyuki Kaneko, Shinzo Kekkan Kenkyu Hoho No Kaihatsu (Development in Method of Studying Heart and Blood Vessels) edited by Setsuro Ebashi, page 3, Japan Scientific Societies Press (JSSP) (1983)), the tissue fragments were digested directly with a protease such as trypsin or collagenase. In the method of the invention, on the other hand, enzyme digestion was carried out after the tissue fragments were washed 4 to 5 times with a phosphate-buffered physiological saline to remove a majority of blood-derived non-heart muscle cells. Removal of blood-derived non-heart muscle cells by washing was carried out by placing the heart tissue fragments on a cell strainer (Falcon) and washing it with a phosphate-buffered physiological saline. For enzyme digestion, the tissue fragments from the 10 newborns were treated for 15 minutes with 5 ml enzyme solution (solution of 1.25 mg trypsin (Difco) and 0.25 mg collagenase (Sigma) per ml of PBS(-)), and 2.5 ml enzyme solution was added additionally twice at a 15-minutes interval, and the tissue fragments were kept at 37°C under stirring with a stirrer. In this process, individual cells were separated from the tissue fragments. After the reaction was finished, Medium 199 (Gibco) containing 10 % fetal calf serum (Biowiker) was added in a half volume relative to the enzyme solution, to terminate the enzyme reaction, and the cells were filtered with a cell strainer and then centrifuged at 400×g for 5 minutes, whereby the cells were collected. Then, the cells from the 10 newborns were suspended in 50 ml Medium 199 containing 10 % fetal calf serum, plated in 100-ml Petri dishes (Iwaki) in a volume of 10 ml/disk and cultured for 1 hour in a CO₂ incubator set at 5 % CO₂, 37°C. The floating cells were recovered, filtered with a cell strainer and centrifuged at 400×g for 5 minutes to collect the heart muscle cells.

Then, the heart muscle cells from the 10 newborns were suspended in 2 ml low-isotonic solution (solution prepared by dissolving 8.29 g NH₄Cl, 1.0 g KHCO₃, 37 mg EDTA/2Na in 1 L of water) and left for 3 minutes, whereby erythrocytes were disrupted. 10 ml Medium 199 containing 10 % fetal calf serum was added thereto, and the heart muscle cells were collected by centrifugation at 400×g for 5 minutes. The cell pellet was suspended in Medium 199 containing 10 % fetal calf serum and then filtered with a cell strainer. In the conventional method, this low-isotonic hemolysis procedure was not conducted.

After 0.3 % trypan blue was added to, and mildly mixed with, an aliquot of the resultant cell suspension, the number of heart muscle cells was counted with an erythrocyte counting plate. In the conventional method, 7.8×10⁶ heart muscle cells were obtained from the hearts of 10 newborns, but contaminated with erythrocytes about 7 times as high as the heart muscle cells. In the method of the invention, on the other hand, 1.4×10⁷ heart muscle cells almost free of erythrocytes were obtained from the hearts of 10 rat newborns.

The cells prepared in each of the conventional method and the method of the invention were suspended at a density of 4×10⁵ cells/ml in Medium 199 containing 10 % fetal calf serum and then cultured in a CO₂ incubator set at 5 % CO₂, 37°C, and the adherent cells were recovered, and the purity of the heart muscle cells were confirmed by flow cytometry (FACSan, produced by Becton Dickinson). The 2×10⁶ cells were washed twice with a phosphate-buffered physiological saline and fixed with 70 % ethanol. The fixed cells were washed twice with a wash buffer (phosphate-buffered physiological saline containing 0.2 % fetal calf serum and 0.1 % NaN₃) and then suspended in 200 µl wash buffer, and after a mouse anti α-actinin antibody (Sigma) was added thereto, the cells were kept at 4°C for 30 minutes in the dark. The cells were washed twice with the wash buffer and then suspended in 200 µl wash buffer, and after a FITC-labeled anti-mouse antibody (Jackson Immuno Research Laboratory) was added thereto, the cells were kept at 4°C for 30 minutes in the dark. The cells were washed twice with the wash buffer, suspended in 500 µl wash buffer and analyzed by flow cytometry. The results are shown in Fig. 1. The purity of the heart muscle cells was about 70 % in the conventional method and about 90 % in the method of the invention.

### Example 2: Comparison of Sensitivity in Detection of Inhibitory Action of Leukemia Inhibitory Factor on Apoptosis

Using the heart muscle cells of rat newborns obtained by the present method of preparing heart muscle cells as described in Example 1, their apoptosis was induced by removing serum from the culture, and the inhibitory action of recombinant human leukemia inhibitory factor (hLIF, produced by Peprotech) on the apoptosis was detected by the conventional method with MTT as the coloration substrate and by the present method of detecting apoptosis with WST-8 or MTT as the coloration substrate, and the sensitivity of the two methods was compared.

First, the muscle cells obtained in the method of preparing heart muscle cells according to the invention were suspended at a density of 3×10⁵ cells/ml in Medium 199 containing 10 % fetal calf serum, put onto a 96-wells plate in a volume of 0.1 ml/well, and then cultured for 1 day in a CO₂ incubator set at 5 % CO₂, 37°C. The plate was stirred with a micromixer (Taiyo Kagaku Kogyo Co., Ltd.) and the medium was exchanged 3 times with serum-free Medium 199, followed by adding 0 to 1000 U/ml hLIF. The culture was incubated for additional 4 days to induce apoptosis. In the conventional method, the number of viable cells in the culture was measured directly with MTT as the coloration substrate. In the method of the invention, on the other hand, fetal calf serum was added thereto at a concentration of 10 %, and the cells were further cultured for about 17 hours in a CO₂ incubator set at 5 % CO₂, 37°C, and the number of viable cells was determined by using WST-8 or MTT as the coloration substrate.

Fig. 2 shows the results of measurement of the inhibitory activity of hLIF at various concentrations on apoptosis of heart muscle cells by the conventional method with MTT as the coloration substrate and by the present method of detecting apoptosis with WST-8 or MTT as the coloration substrate. In the conventional method, the tendency of inhibition of apoptosis of heart muscle cells by hLIF can be seen, but the increase in the average absorbance by adding 1000 U/ml hLIF, as compared with the control group to which hLIF had not been added, was as very low as 0.03 or less. This is below the limit of detection by the measuring device, and the conventional method cannot be applied to screening with a large number of samples. In the present method of detecting apoptosis with MTT as the coloration substrate, on the other hand, the average absorbance of the control group to which hLIF had not been added was as high as 0.1 or more, and by adding 1000 U/ml hLIF, the absorbance was further increased by nearly 0.1, and thus the method of the invention is by far more accurate than the conventional method. This method would be applicable to screening where the absorbance or turbidity of a sample itself is not troublesome or for the purpose of searching for a sample indicating a very strong apoptosis-inhibiting activity equal to or higher than that of 1000 U/ml hLIF. In actual screening, however, the detection sensitivity of a measuring device and noises attributable to the absorbance or turbidity of a sample itself can be often troublesome, and it is thus desirable that an increase of 0.1 or more in absorbance is used as selection reference for correctly selecting an active sample. It was found that in the method of detecting apoptosis according to this invention in the case where WST-8 was used as the coloration substrate, the sensitivity was further improved, the average absorbance of the control group to which hLIF had not been added was 0.2 or more, and by adding 1000 U/ml hLIF, the average absorbance was increased by 0.3 or more, while noises were tolerable.

From the foregoing, it was found that the method of detecting apoptosis according to this invention can be used to detect not only the activity of a sample exhibiting, at an equal level to 1000 U/ml hLIF, an inhibitory action on apoptosis of heart muscle cells, an agonistic action on gp130-mediated receptors or an enhancing action on heart muscle cell-protective signals, but also the activity of a mixture of many samples by establishing suitable selection reference.

### Example 3: Practice of Measurement Dealing with A Large Number of Samples

It was examined whether screening dealing with a large number of samples was feasible or not by a method of detecting apoptosis, which comprises culturing heart muscle cells on a 96-well plate obtained by the present method of preparing heart muscle cells, then removing serum from the culture to induce apoptosis, also adding 1000 U/ml hLIF to inhibit the apoptosis, and detecting the apoptosis with WST-8 as the coloration substrate.

First, the heart muscle cells obtained in the method described in Example 1 were suspended at a density of 3×10⁵ cells/ml in Medium 199 containing 10 % fetal bovine serum, and put in a volume of 0.1 ml/well onto each well of 2nd to I 1th rows on each of three 96-wells plates. The 1st and 12th rows were used as the blank free of the cells. The cells were cultured for 1 day in a CO₂ incubator set at 5 % CO₂, 37°C, and then stirred with a micromixer (Taiyo Kagaku Kogyo Co., Ltd.) and then the medium was exchanged 3 times with serum-free Medium 199 to remove serum. Then, 1000 U/ml hLIF was added to each well on the 2nd to 6th rows of each plate. The cells were cultured for 4 days to induce apoptosis, and fetal calf serum was added at a concentration of 10 %, and the cells were cultured for additional 17 hours in a CO₂ incubator set at 5 % CO₂, 37°C, and then the number of viable cells was counted with WST-8 as the coloration substrate.

The results are shown in Table 1.

The average absorbance of the control group not containing hLIF was 0.22±0.039 on plate 1,0.15±0.028 on plate 2 and 0.13±0.020 on plate 3, and by adding 1000 U/ml hLIF, the absorbance was increased to 0.54±0.055, 0.45±0.035 and 0.43±0.059, respectively. In actual screening, noises attributable to the absorbance or turbidity of a sample itself can be troublesome, and it is thus desired that an increase of 0.1 or more in absorbance be used as selection reference for correctly selecting an active sample. It was found that by this method, where the activity of increasing the absorbance by 0.15 or more relative to that of the control group is selected as selection reference, 120 samples exhibiting, at a level equal to 1000 U/ml hLIF, an inhibitory action on apoptosis of heart muscle cells, an agonistic action on gp130-mediated receptors and an enhancing action on heart muscle cell-protective signals can be selected from 240 samples.

**[Table 1]**

| Plate. 1 | | hLIF 1000U/ml | | | | | hLIF 0U/ml | | | | | |
|---|---|---|---|---|---|---|---|---|---|---|---|---|
| | 1 | 2 | 3 | 4 | 5 | 6 | 7 | 8 | 9 | 10 | 11 | 12 |
| A | | 0.495 | 0.453 | 0.613 | 0.491 | 0.607 | 0.197 | 0.164 | 0.172 | 0.164 | 0.311 | |
| B | | 0.563 | 0.437 | 0.556 | 0.503 | 0.538 | 0.210 | 0.171 | 0.180 | 0.185 | 0.192 | |
| C | | 0.577 | 0.423 | 0.528 | 0.535 | 0.525 | 0.218 | 0.230 | 0.162 | 0.231 | 0.206 | |
| D | | 0.547 | 0.440 | 0.532 | 0.529 | 0.514 | 0.193 | 0.184 | 0.163 | 0.246 | 0.182 | |
| E | | 0.498 | 0.458 | 0.536 | 0.438 | 0.493 | 0.200 | 0.230 | 0.163 | 0.282 | 0.219 | |
| F | | 0.472 | 0.436 | 0.628 | 0.500 | 0.564 | 0.180 | 0.224 | 0.187 | 0.219 | 0.173 | |
| G | | 0.597 | 0.555 | 0.561 | 0.548 | 0.629 | 0.272 | 0.299 | 0.228 | 0.285 | 0.262 | |
| H | | 0.684 | 0.633 | 0.684 | 0.612 | 0.644 | 0.367 | 0.267 | 0.261 | 0.276 | 0.239 | |
| Plate. 2 | | | | | | | | | | | | |
| | 1 | 2 | 3 | 4 | 5 | 6 | 7 | 8 | 9 | 10 | 11 | 12 |
| A | | 0.424 | 0.447 | 0.548 | 0.531 | 0.490 | 0.166 | 0.115 | 0.131 | 0.124 | 0.197 | |
| B | | 0.480 | 0.469 | 0.475 | 0.467 | 0.405 | 0.137 | 0.125 | 0.114 | 0.088 | 0.124 | |
| C | | 0.482 | 0.491 | 0.373 | 0.500 | 0.432 | 0.194 | 0.120 | 0.129 | 0.149 | 0.114 | |
| D | | 0.459 | 0.457 | 0.436 | 0.415 | 0.367 | 0.152 | 0.108 | 0.110 | 0.151 | 0.118 | |
| E | | 0.406 | 0.476 | 0.436 | 0.457 | 0.458 | 0.182 | 0.140 | 0.149 | 0.156 | 0.195 | |
| F | | 0.446 | 0.461 | 0.455 | 0.391 | 0.461 | 0.151 | 0.157 | 0.122 | 0.113 | 0.180 | |
| G | | 0.416 | 0.406 | 0.422 | 0.403 | 0.486 | 0.202 | 0.155 | 0.145 | 0.141 | 0.215 | |
| H | | 0.460 | 0.420 | 0.390 | 0.401 | 0.560 | 0.235 | 0.220 | 0.185 | 0.161 | 0.190 | |
| Plate. 3 | | | | | | | | | | | | |
| | 1 | 2 | 3 | 4 | 5 | 6 | 7 | 8 | 9 | 10 | 11 | 12 |
| A | | 0.353 | 0.345 | 0.403 | 0.369 | 0.405 | 0.124 | 0.126 | 0.131 | 0.167 | 0.127 | |
| B | | 0.453 | 0.299 | 0.362 | 0.321 | 0.363 | 0.116 | 0.102 | 0.105 | 0.100 | 0.109 | |
| C | | 0.395 | 0.364 | 0.382 | 0.387 | 0.341 | 0.102 | 0.120 | 0.102 | 0.147 | 0.104 | |
| D | | 0.419 | 0.356 | 0.421 | 0.377 | 0.374 | 0.118 | 0.124 | 0.114 | 0.107 | 0.117 | |
| E | | 0.488 | 0.419 | 0.386 | 0.369 | 0.494 | 0.162 | 0.124 | 0.129 | 0.162 | 0.116 | |
| F | | 0.451 | 0.435 | 0.485 | 0.580 | 0.584 | 0.137 | 0.139 | 0.161 | 0.161 | 0.133 | |
| G | | 0.459 | 0.433 | 0.484 | 0.594 | 0.552 | 0.146 | 0.189 | 0.129 | 0.161 | 0.165 | |
| H | | 0.489 | 0.428 | 0.565 | 0.577 | 0.606 | 0.149 | 0.165 | 0.141 | 0.165 | 0.175 | |

### Example 4: Detection of Inhibitory Action of Insulin Like Growth Factor-1 on Apoptosis

Insulin like growth factor-1 (hIGF-1) is known to inhibit apoptosis of heart muscle cells by activating Akt (Fujio Y. et al., Circulation, vol. 101, p. 660, 2000). Accordingly, the inhibitory action of recombinant human IGF-1 (hIGF-1, produced by Peprotech) on heart muscle cell apoptosis induced by removing serum from a culture was measured by the present method of detecting apoptosis with WST-8 as a coloration substrate.

First, heart muscle cells obtained by the method of preparing heart muscle cells according to the invention were suspended at a density of 3 ×10⁵ cells/ml in Medium 199 containing 10 % fetal calf serum and cultured for 1 day in a CO₂ incubator set at 5 % CO₂, 37°C. The cells were stirred with a micromixer (Taiyo Kagaku Kogyo Co., Ltd.) and the medium was exchanged 3 times with serum-free Medium 199, and hIGF-1 was added thereto at a final concentration of 0 to 100 nM. The culture was cultured for additional 4 days to induce apoptosis. Fetal calf serum was added thereto at a concentration of 10 %, and the cells were further cultured for about 17 hours in a CO₂ incubator set at 5 % CO₂, 37°C, and the number of viable cells was determined with WST-8 as the coloration substrate.

Fig. 3 shows the results of measurement of the inhibitory activity of hIGF-1 at various concentrations on apoptosis of heart muscle cells. As shown in the result, this method can also be used to search for the compound showing a protective action on heart muscles in a mechanism different from that of gp130 signal.

### Reference Example 1: 2-(2-pyridyl)-4H-1,3-benzothiazin-4-one (Compound 1)

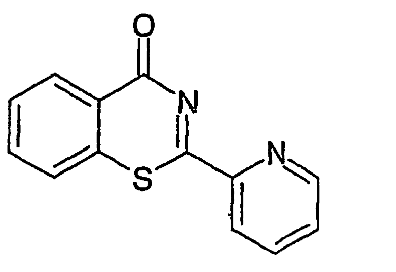

Methyl thiosalicylate (1.6 g, 9.51 mM) and 2-cyanopyridine (1.0 g, 9.60 mM) were dissolved in toluene (2 ml), and triethylamine (2 ml, 14.4 mM) was added thereto, and after the mixture was heated under reflux for 8 hours, the toluene was distilled away. Ethanol was added to the residue, and precipitates were separated by filtration to give crude crystals (1.7 g). This product was purified by silica gel column chromatography (hexane : chloroform = 5 : 1 → chloroform) to give the title compound as crystals (1.0 g, 43.4 %).
Elemental analysis of C₁₃H₈N₂OS
Calculated (%): C: 64.98, H: 3.36, N: 11.66
Found (%): C: 64.93, H: 3.31, N: 11.59
¹H-NMR (CDCl₃)δ: 7.50-7.75(m, 4H), 7.85-8.00(m, 1H), 8.50-8.60(m,2H), 8.70-8.80(m, 1H)
IR (KBr): 1660cm⁻¹
Melting point: 173.5-174.3°C

### Example 5

Tablets were produced in a usual manner from Compound 1 (100 mg), lactose (165 mg), corn starch (25 mg), polyvinyl alcohol (4 mg) and magnesium stearate (1 mg).

### Industrial Applicability

Highly pure heart muscle cells can be produced easily in a large amount by the method of preparing heart muscle cells according to this invention. Further, apoptosis of the heart muscle cells can be detected efficiently and highly sensitively by the method of detecting apoptosis according to this invention.

Further, the screening method of the invention can be used for the efficient and highly sensitive screening of compounds inhibiting apoptosis of heart muscle cells or salts thereof, and can deal with a large number of samples for screening rapidly and efficiently, whereby compounds having an inhibitory action on apoptosis of heart muscle cells, compounds having an agonistic action on gp130-mediated receptors or compounds having an enhancing action on heart muscle cell-protective signals can be obtained. Such compounds are useful as prophylactic and/or therapeutic agents for heart diseases.

## Claims

1. A method of screening for a compound inhibiting apoptosis or a salt thereof comprising comparing the number of viable cells in case (i) and case (ii)
(i) after serum is added to a culture of heart muscle cells having apoptosis induced in the presence of a test compound by culturing the cells in a serum-free medium, culturing the cells and determining the number of viable heart muscle cells, and
(ii) after serum is added to a culture of heart muscle cells having apoptosis induced in the absence of a test compound by culturing the cells in a serum-free medium, culturing the cells and determining the number of viable heart muscle cells,
wherein the heart cells are obtained by a method in which after non-heart muscle cells are removed fragmented heart tissue, the heart tissue is digested with a protease and that after digestion erythrocytes are removed.

2. The method according to claim 1 wherein the non-heart muscles are removed by washing the fragmented heart tissue with a phosphate buffered physiological saline.

3. The method according to claim 1 wherein the erythrocytes are removed by hemolysis treatment.

4. The method according to claim 1 wherein the number of viable heart muscle cells is determined by measuring an intracellular dehydrogenase activity.

5. The method according to claim 4 wherein the culture time is 6 to 30 hours.

6. The detection method according to claim 5 wherein the intracellular dehydrogenase activity is measured by using MTT or WST-8.

7. A method of preparing primary heart muscle cells, **characterized in that** after non-heart muscle cells are removed from fragmented heart tissue, the heart tissue is digested with a protease.

8. The preparative method according to claim 7, **characterized in that** the non-heart muscle cells are removed by washing the fragmented heart tissue with a phosphate-buffered physiological saline.

9. A method of preparing primary heart muscle cells, **characterized in that** after fragmented heart tissue is digested with a protease, erythrocytes are removed.

10. The preparative method according to claim 9, **characterized in that** erythrocytes are removed by hemolysis treatment.

11. A method of preparing primary heart muscle cells, **characterized by** hemolysis treatment of heart muscle cells isolated from heart tissue.

12. A method of preparing primary heart muscle cells, **characterized in that** after non-heart muscle cells are removed from fragmented heart tissue, the heart tissue is digested with a protease, and then erythrocytes are removed.

13. The preparative method according to claim 12, **characterized in that** the fragmented heart tissue is washed with a phosphate-buffered physiological saline to remove non-heart muscle cells, and the heart tissue digested with a protease is subjected to hemolysis treatment to remove eryhrocytes.

14. A method of detecting apoptosis of heart muscle cells, **characterized in that** after serum is added to a culture of heart muscle cells having apoptosis induced by culturing the cells in a serum-free medium, the cells are cultured, and the number of viable heart muscle cells is determined.

15. The detection method according to claim 14, wherein the number of viable heart muscle cells is determined by measuring an intracellular dehydrogenase activity.

16. The detection method according to claim 14, wherein the culture time after addition of serum is about 6 to 30 hours.

17. The detection method according to claim 15, wherein the intracellular dehydrogenase activity is measured by using MTT or WST-8.

18. The detection method according to claim 14, wherein the primary heart muscle cells obtained by the preparative method described in any one of claims 7 to 13 are cultured in a serum-free medium.

19. A method of screening a compound inhibiting apoptosis or a salt thereof, **characterized in that** by the detection method described in claim 18, the number of viable cells in a case (i) where apoptosis is induced in the presence of a test compound is compared with that in a case (ii) where apoptosis is induced in the absence of a test compound.

20. . An inhibitor of apoptosis of heart muscle cells, comprising a compound obtained by the screening method described in claim 19 or a salt thereof.

21. A gp130-mediated receptor agonist, comprising a compound obtained by the screening method described in claim 19 or a salt thereof.

22. An enhancer of heart muscle cell-protective signal, comprising a compound obtained by the screening method described in claim 19 or a salt thereof.

23. A prophylactic and/or therapeutic agent for heart diseases, comprising the heart muscle cell apoptosis inhibitor described in claim 20, the gp130-mediated receptor agonist described in claim 21 or the heart muscle cell-protective signal enhancer described in claim 22.

24. A method of preventing and treating heart diseases, **characterized in that** an effective dose of the heart muscle cell apoptosis inhibitor described in claim 20, the gp130-mediated receptor agonist described in claim 21 or the heart muscle cell-protective signal enhancer described in claim 22 is administered to mammals.

25. Use of the heart muscle cell apoptosis inhibitor described in claim 20, the gp130-mediated receptor agonist described in claim 21 or the heart muscle cell-protective signal enhancer described in claim 22 for producing a prophylactic and/or therapeutic agent for heart diseases.
